Europäisches Patentamt

**European Patent Office**

Office européen des brevets

(11) Publication number: **0 274 432**
**A1**

# EUROPEAN PATENT APPLICATION

(21) Application number: **88300092.9**

(22) Date of filing: **07.01.88**

(51) Int. Cl.⁴: **C 07 C 7/152**
**C 10 G 29/08, C 10 M 175/00,**
**C 10 M 177/00**
**// B01D27/02**

(30) Priority: **07.01.87 GB 8700240**

(43) Date of publication of application:
**13.07.88 Bulletin 88/28**

(84) Designated Contracting States:
**AT BE CH DE ES FR GB GR IT LI LU NL SE**

(71) Applicant: **EXXON CHEMICAL PATENTS INC.**
**1900 East Linden Avenue**
**Linden New Jersey 07036 (US)**

(72) Inventor: **Brownawell, Darrell William**
**320 Scotch Plains**
**New Jersey 07076 (US)**

**Doyle, Gerald**
**Box 20, RD No. 1**
**Whitehouse Station New Jersey 08889 (US)**

**Hoel, Elvin Lynn**
**209 Florence Avenue**
**Westfield New Jersey 07090 (US)**

(74) Representative: **Bawden, Peter Charles et al**
**EXXON CHEMICAL LIMITED EXXON CHEMICAL**
**TECHNOLOGY CENTRE PO Box 1**
**Abingdon Oxfordshire OX13 6BB (GB)**

The title of the invention has been amended (Guidelines for Examination in the EPO, A-III, 7.3).

(54) **Process for the removal of polynuclear aromatic compounds from hydrocarbon streams.**

(57) This invention concerns the use for the substantial removal of polynuclear aromatic compounds from hydrocarbon streams containing said compounds of a complex of the general formula (Cu R SO₃)₂ Ar in which R is a hydrocarbon or substituted hydrocarbon group and Ar is an aromatic group. In the process of removing the polynuclear aromatic compounds the stream can be passed through a filter element comprising the complex.

EP 0 274 432 A1

**0 274 432**

## Description

Polynuclear Aromatics

The present invention relates to the removal of polynuclear aromatic compounds from hydrocarbon streams, particularly from used lubricating oils and gaseous and liquid refinery feeds.

Polynuclear aromatic compounds especially those containing three or more aromatic nuclei are frequently present in relatively small quantities in hydrocarbon streams, which given their carcinogenity, leads to health and environmental concerns. This is particularly evident in used lubricating oil from gasoline engines where the high temperatures during engine operation tend to promote the formation of polynuclear aromatics in the oil leading to concentrations higher than 100 parts per million rendering disposal of the used oil hazardous.

We have now found that certain complexes are particularly useful for the removal of these polynuclear aromatics. The present invention therefore provides the use of a complex of the general formula $(Cu\,R\,SO_3)_2$. Ar (in which R is hydrocarbon or substituted hydrocarbon group and Ar is an aromatic group) for the substantial removal of polynuclear aromatic compounds from hydrocarbon streams containing them. Also according to this invention polynuclear aromatic compounds are substantially removed from a liquid or gaseous hydrocarbon stream containing said polynuclear aromatic compounds by a process comprising contacting said stream with a complex of the formula $(Cu\,R\,SO_3)_2$, Ar where R is a hydrocarbon group or a substituted hydrocarbon group and Ar is an aromatic group, and collecting said hydrocarbon stream substantially depleted in said polynuclear aromatic compounds.

Complexes of the general formula are known from United States patents 3634530, 3647840 and 3763200 as well as from the JCS10 Comm. of 1972 p559 and of 1973 p12. These references suggest that the complexes may be used in the separation of aromatic compounds from mixed aromatic streams, especially for the separation of xylene isomers. There is, however, no suggestion of the selectivity of these complexes to the polynuclear aromatics of the type with which the present invention is concerned and which presents the health and toxicological problems.

We have found that the complexes of the present invention which are usually complexes with mono-nuclear aromatics will readily undergo exchange reactions between the mono-nuclear aromatic and the polynuclear which it is designed to remove from the hydrocarbon feed and the use of these materials has in laboratory experiments reduced the content of polynuclear aromatics such as phenanthrene, pyrene, 1,2-benzanthrene with model complexes form 8,000 ppm to $\geq$ 100 ppm by passing compositions containing these materials over the defined complexes for a period of from 1 to 4 hours.

We find that the removal of the polynuclear aromatics may be conveniently achieved by impregnating a substrate which is porous to the flow of the particular hydrocarbon stream with the defined complex so that the hydrocarbon stream passes across the substrate so depositing the unwanted polynuclear aromatics on the substrate leaving the stream downstream of the substrate depleted in these unwanted materials. The choice of the substrate will, of course, depend on the nature of the hydrocarbon stream to be treated and the type of operation with which one is concerned.

For example, in the treatment of used lubricating oil the substrate may be in the form of a filter positioned within the lubricating system of the vehicle so that the polynuclear aromatics are deposited on the filter during the normal flow of the lubricant from the system and may therefore by removed and readily disposed of simply by removal of a small filter. This is far simpler than resorting to present techniques with the problem of disposing of large volumes of lubricating oil having a high polynuclear aromatic content.

Similarly, the technique may be used to treat both liquid and gaseous hydrocarbon feeds in refinery streams including all crude oil feed to the refinery and all of the gaseous and liquid feed streams produced in the refinery where again the substrate may be in the form of a filter placed in the flow path of the particular stream, although of course the necessary strength and porosity of the filter will vary depending as to whether the feed is liquid, and if so, of its viscosity, and also whether the stream is gaseous.

Similarly the conditions prevalent at the position where the filter is located such as temperature and pressure will determine the nature of the filter material.

Suitable substrates comprise attapulgus clay, silica gel, molecular sieves, dolomite clay, alumina or zeolite. Of course it may be necessary to provide a container to hold the impregnated substrate. Suitable filters may be circular masses of impregnated substrate supported on wire gauze. Alternatively the filters could comprise the solid complex held in pockets of filter paper.

The complexes used in the present invention are known and may conveniently be prepared by the techniques described in United States patents 3634530, 3647840 and 3763200.

The complexes used to remove polynuclear compounds are of the formula $(Cu\,R\,SO_3)_2$ Ar where R and Ar are as previously defined. In these complexes which are complexes of a cuprous sulphonate salt, copper is in the monovalent state.

The sulphonic acids from which the cuprous salts are defined can be represented by the structural formula $RSO_3H$, wherein R preferably contains from 1 to 20 carbon atoms and preferably contains the substituents chlorine or fluorine or combinations thereof. Preferably, the R group will contain from 1 to 10 carbon atoms. Thus, the R group on the sulphonic acid starting material and the R group in the cuprous sulphonate salt product can be hydrocarbon, flurocarbon, chlorocarbon, flurohydrocarbon, chlorohydrocarbon and flurochlorohydrocarbon. Non-limiting examples of the sulphonic acid starting materials include the alkane and

2

substituted alkane sulphonic acid, methane sulphonic acid, chloromethane sulphonic acid, chlorofluoromethane sulphonic acid, difluormethane sulphonic acid, trifluromethane sulphonic acid, ethane sulphonic acid, 2-chloroethane sulphonic acid, pentachloroethane sulphonic acid, isobutane sulphonic acid, propane sulphonic acid, heptachloropropane sulphonic acid, n-heptane sulphonic acid, decane sulphonic acid, n-eicosane sulphonic acid cyclohexane sulphonic acid, 2-chloro-4-fluoro-6-ethylbenzene sulphonic acid, and the aryl and substituted aryl sulphonic acids, benzene sulphonic acid, para-toluene sulphonic acid, para-chlorobenzene sulphonic acid, 2,4,6-trifluorbenzene sulphonic acid, β-naphthalene sulphonic acid and parabutylbenzene sulphonic acid.

Alternatively R can be a per fluoro polymer group eg a polyfluoropolyethylene group or for example a polyhydrocarbon such as polystyrene.

The complexing agent that is a portion of the cuprous sulphonate salt complexes (Ar) can be a hydrocarbon aromatic compound, preferably containing up to 10 carbon atoms per molecule. Suitable aromatic compounds are benzene, toluene, o-xylene, m-xylene, p-xylene, ethybenzene, styrene 1,3,5-trimethylbenzene, naphthalene, -ethylnaphthalene, and the like.

Alternatively Ar could be an alkyl phenol or an alkyl phenol sulphide, for example nonyl phenol or nonyl phenol sulphide.

The preferred complexes are those where R is a fluoro group, especially trifluoromethane - CF$_3$, or pentafluorethane-CF$_5$ and where Ar is an aryl compound such as benzenen or toluene. The especially preferred complex is (CuCF$_3$SO$_3$) C$_6$H$_6$. Another suitable example is the cuprous salt of a polymeric sulphonic acid complex, eg the cuprous salt of polyfluoroethylene sulphonic acid complexed with benzene, supported on alumina.

It is highly desirable that both the complex used in the filter and the complex formed after reaction with the polynuclear aromatic compound are insoluble or nearly insoluble in the feedstream, eg used engine oil, being treated.

The amount of complex required will vary according to the concentration of the polynuclear aromatic compounds in the feedstream but when using for example the benzene or toluene complex of copper trifluoro methane sulphonic acid [ (CuCF$_3$SO$_3$)$_2$ C$_6$H$_6$ or (CuCF$_3$SO$_3$)$_2$ C$_6$H$_5$CH$_3$] about 100 to 120 ppm of the complex can remove about 65 to 75 ppm of polynuclear aromatic compounds from the feedstream, eg used engine oil. Used engine oils usually contain 10 to 1000 eg 10 to 100 ppm, of polynuclear aromatic compounds and other feedstreams will have comparable amounts. The removal or substantial removal of polynuclear aromatic compounds from the feedstream will usually take up to a week or two weeks by the process of this invention.

When solid complexes are used to make up the filter it is preferred that the complex is mixed with another compound for example nonyl phenol sulphide. Such compounds re included to give antioxidant properties to the oil emerging from the filter.

By the process of this invention polynuclear aromatic compounds especially those with three or more rings can be substantially removed (ie a reduction of 60% to 80%) from liquid or gaseous hydrocarbon streams.

Examples of trinuclear aromatic compounds which are removed are phenanthrene, anthracene and 9,10-dihydro anthracene.

Examples of tetranuclear aromatic compounds which are removed are pyrene, 1,2-benzanthracene, chrysene, tetracene and fluoranthrene whilst examples of pentanuclear aromatic compounds which are removed are dibenzanthracene, benzo(e)pyrene, benzo(b)fluoranthene, benzo(k)fluoranthene and benzo(a)pyrene. Examples of hexanuclear aromatic compounds which are removed are benzo(phi)perylene and coronene.

Example 1

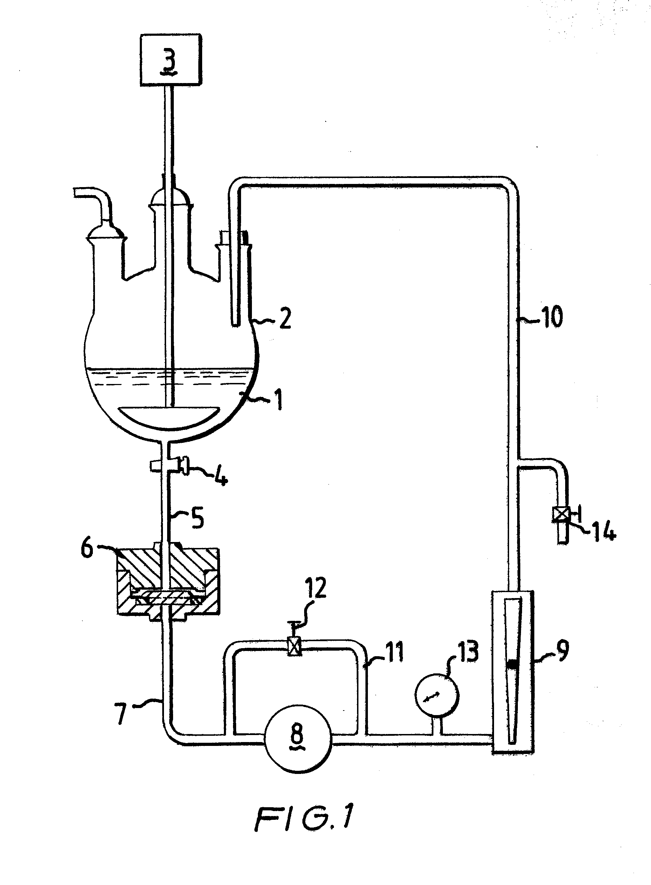

In this Example laboratory apparatus was used for testing the removal of polynuclear aromatics from used motor oils and the apparatus used is illustrate in Figure 1.

Referring to Figure 1 the used motor oil 1 was placed in a 250 ml flask 2 provided with a stirrer 3. Tubing 5 provided with a tap 4 connects the bottom of the flask 2 with a teflon filter unit 6. Connected downstream of this filter unit 6 is tubing 7 provided with a pump 8 connecting to a rotameter 9 to measure the rate of flow of oil. Tubing 10 connects the rotameter 9 with the flask 2. The pump 8 is provided by with a bypass 11 having a tap 12 and a gauge 13 can measure the oil pressure in tubing 7. Finally there is a drain tap 14.

In the filter the copper complex was sandwiched between two sheets of commercial oil filter paper. The copper complex was the benzene complex of copper trifluoro methane sulphonic acid (Cu CF$_3$ SO$_3$)$_2$C$_6$H$_6$.

The composition of the used lubricating oil before passing through the filter was as given in column 1 of Table 1.

The oil treated for approximately 100 hours (Expt 1) and approximately 100 hours (Expt 2) by continuously circulating it in the apparatus illustrated in Figure 1. The distribution of polynuclear aromatics in the oil was measured after the two treatments and the results obtained are given in columns 2 and 3 of Table 1. Column 4 gives the percentage reduction of polynuclear aromatics in Experiment 2. It can be seen that a considerable reduction of the content of polynuclear aromatics is achieved.

Table 1

0 274 432

| Compound | Untreated Oil | Treated Oil | | % Reduction in Expt 2 |
|---|---|---|---|---|
| | | Expt 1 | Expt 2 | |
| Fluoranthene | 10.4 | 9.3 | 6.6 | 37% |
| Pyrene | 22.6 | 22.0 | 19.3 | 15% |
| Benzo(a)anthracene | 8.7 | 6.6 | 5.1 | 41% |
| Chrysene | 9.6 | 7.4 | 5.6 | 42% |
| 4-Ring Aromatic | 51.3 | 45.3 | 36.6 | 29% |
| | | | | |
| Benzo(e)pyrene | 9.9 | 7.0 | 5.9 | 44% |
| Benzo(b)fluoranthene | 10.4 | 8.2 | 8.2 | 21% |
| Benzo(k)fluoranthene | 2.2 | 1.3 | 1.4 | 36% |
| Benzo(a)pyrene | 5.0 | 2.7 | 2.3 | 54% |
| 5-Ring Aromatic | 27.5 | 19.2 | 17.8 | 35% |
| | | | | |
| Benzo(phi)perlene | 15.7 | 13.5 | 12.0 | 24% |
| Coronene | 6.6 | 4.7 | 4.7 | 29% |
| 6+Ring Aromatic | 22.3 | 18.2 | 16.7 | 25% |
| | | | | |
| TOTAL | 101.1 | 82.7 | 71.1 | -30% |

Example 2

In this Example a solution of approximately 0.08g phenanthrene (a typical trinuclear aromatic) in 10g methylcyclohexane was stirred with approximately 1g $(CuCF_3SO_3)_2$ $C_6H_6$ in a flask sealed with a rubber septum. After addition of the copper complex to the phenanthrene solution the flask was sealed and samples of the clear liquid were withdrawn by a syringe. The samples were analysed by gas chromatography using a 10 ft 1/8″ diameter stainless steel column packed with 20% SP2100 on supelcoport. From an initial concentration of approximately 8000 ppm the phenanthrene concentration was reduced to 3600 ppm after 38 min, 1900 ppm after 100 minutes and less than 70 ppm after 234 minutes.

Example 3

An experiment similar to that described in Example 2 was carried out using a solution of approximately 0.07g pyrene (a typical four ringed aromatic) in 8g methylcyclohexane with 1g $(CuCF_3SO_3)_2C_6H_6$. From an initial concentration of 9390 ppm the pyrene was reduced to 5090 ppm after 451 minutes and to less than 40 ppm after 1347 minute.

Example 4

In this Example a solution of approximately 0.06g of 1,2-benzanthracene (a four ring aromatic) in 9.7g methylcyclohexane was stirred with 1g $(CuCF_3SO_3)_2.C_6H_6$. The concentration of 1,2-benzanthracene was reduced to trace levels (<100 ppm) in 150 minutes.

Example 5

This example is similar to Example 2 except that $(CuCF_3SO_3)_2.C_7H_8$ was used in place of $(CuCF_3SO_3)_2.C_6H_6$. From an initial concentration of approximately 8300 ppm phenanthrene the concentration was reduced to 1530 ppm after 30 minutes and to less than 40 ppm after 58 minutes.

Example 6

In this Example five experiments were carried out and details of the starting oil, the amount of complex used, the hours treated and the ratio of ppm of copper to oil treated are given in Table 2.

## Table 2

| Expt | Type and Amount of Oil | Amount and Details of Complex | Hours Treated | Ratio of Cu to Oil Treated (ppm) |
|---|---|---|---|---|
| 1 | 64g used engine oil | 0.52g Copper trifluoro methane sulphonic acid (CTFS) | 71 | 2000 |
| 2 | 63.3g used engine oil | 2.94g CTFS as pellets | 143 | - |
| 3 | 52.7g used engine oil | 0.56g CTFS on 2.28g alumina | 112 | 2500 |
| 4 | 51.8g used engine oil | 10.11g cuprous salt of sulphonated Teflon (fully fluorinated polyethylene) | 140 | 2300 |
| 5 | 50g mineral oil (Stanco 150) and polycyclics[1] | 1.37g CTFS on 4.09g alumina | 89 | 6600 |

(1) polycyclics 500 ppm phenanthrene plus 500 ppm pyrene.

The results are shown in Table 3.

0 274 432

Table 3

Removal of Polynuclear Aromatics

| Compound | Untreated Oil | Expt 1 | Expt 2 | Expt 3 | Expt 4 | Expt 5 |
|---|---|---|---|---|---|---|
| | | | | Treated Oil | | |
| Fluorene | 3.3 | 3.7 | 3.4 | 3.7 | 3.6 | - |
| Phenanthrene | 21.2 | 23.9 | 19.3 | 20.2 | 17.1 | 181.7 |
| Anthracene | 4.8 | 4.2 | 3.5 | 3.7 | 3.9 | - |
| Reduction 3-ring Ar | | 0% | 11% | 6% | 51% | 64% |
| Fluoranthene | 13.8 | 6.6 | 6.4 | 9.3 | 8.2 | - |
| Pyrene | 22.6 | 19.3 | 17.9 | 16.3 | 16.6 | 154 |
| Triphenylene | 5.3 | 1.7 | 3.1 | 3.2 | 3.1 | - |
| 2, 3-Benzofluorene | 10.5 | 7.5 | 5.9 | 5.5 | 7.0 | - |
| Benz (a) Anthracene | 12.2 | 5.1 | 3.7 | 8.1 | 8.1 | - |
| Chrysene | 9.5 | 5.6 | 5.2 | 6.7 | 6.4 | - |
| Reduction 4-ring Ar | | 38% | 43% | 34% | 42% | 69% |
| Benzo (e) Pyrene | 9.9 | 5.9 | 5.8 | 7.2 | 5.3 | - |
| Benzo (b) Fluranthene | 10.4 | 8.2 | 6.7 | 7.1 | 7.0 | - |
| Perylene | 3.7 | 2.5 | 1.7 | 2.2 | 2.1 | - |
| Benzo (k) Fluoranthene | 2.2 | 1.4 | 1.1 | 1.6 | 1.2 | - |
| Benzo (a) Pyrene | 9.7 | 2.3 | 2.0 | 7.4 | 6.4 | - |
| Dibenzo (ah) Anthracene | 2.2 | 0.5 | 0.4 | 0.5 | 0.4 | - |
| Reduction 5-ring Ar | | 46% | 54% | 32% | 41% | - |
| Benzo (ghi) Perylene | 15.7 | 12.0 | 10.8 | 12.4 | 10.5 | - |
| 1,2,3-Indeno (cd) Pyrene | 6.3 | 5.6 | 4.8 | 3.8 | 4.0 | - |
| Anthanthrene | 3.1 | 1.9 | 1.7 | 2.1 | 1.9 | - |
| Coronene | 6.6 | 4.6 | 3.6 | 4.6 | 3.5 | - |
| Reduction 6+ ring Ar | | 24% | 35% | 28% | 37% | |
| Total PNA Reduction | | 28% | 38% | 27% | 36% | 66% |
| Reduction of 4,5 & 6 ring Ar | | 35% | 44% | 32% | 41% | 69% |

## Claims

1. The use for the substantial removal of polynuclear aromatic compounds from hydrocarbon streams containing said compounds of a complex of the general formula $(Cu\ R\ SO_3)_2$ Ar is which R is a hydrocarbon or substituted hydrocarbon group and Ar is an aromatic group.

2. The use according to claim 1 wherein R contains 1 to 20 carbon atoms.

3. The use according to either of claims 1 and 2 wherein R contains chlorine or fluorine.

4. The use according to any one of the preceding claims wherein Ar is a hydrocarbon aromatic compound containing up to 10 carbon atoms per molecule.

5. The use according to any one of the preceding claims wherein the complex has formula $(CuCF_3SO_3)_2.C_6H_6$ or $(CuCF_3SO_3)_2.\ C_6H_5CH_3$.

6. The use according to any one of claims 1 to 4 wherein the complex is the cuprous salt of polyfluoroethylene sulphonic acid complexed with benzene supported on alumina.

7. A process for the substantial removal of polynuclear aromatic compounds from hydrocarbon streams containing said polynuclear aromatic compounds which comprises contacting said stream with a complex of the general formula $(Cu\ R\ SO_3)_2$ Ar in which R is a hydrocarbon or substituted hydrocarbon group and Ar is an aromatic group and collecting said hydrocarbon stream substantially depleted in said polynuclear aromatic hydrocarbons.

8. A process according to claim 6 wherein R contains 1 to 20 carbon atoms.

9. A process according to either of claims 7 and 8 wherein R contains chlorine or fluorine.

10. A process according to any one of claims 7 to 9 wherein Ar is a hydrocarbon aromatic compound containing up to 10 carbon atoms per molecule.

11. A process according to any one of claims 7 to 10 wherein the complex has the formula $(CuCF_3SO_3)_2.C_6H_6$ or $(CuCF_3SO_3)_2.C_6H_5CH_3$.

12. A process according to any one of claims 7 to 10 wherein the complex is the cuprous salt of polyfluoroethylene sulphonic acid complexed with benzene supported on alumina.

0274432

FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-2 941 018  (R.W. FOREMAN) * Column 1, line 15 - column 2, line 35; column 3, lines 3-12; column 4, lines 34-61 * | 1-5,7-11 | C 07 C    7/152 C 10 G   29/08 C 10 M  175/00 C 10 M  177/00 // B 01 D   27/02 |
| Y | | 6,12 | |
| D,X | US-A-3 763 200  (M.B. DINES) * Abstract; column 1, line 36 - column 2, line 32; column 3, lines 4-42; column 4, lines 1-14,35-50,65 - column 5, line 4; examples 5,6 * | 1-5,7-11 | |
| Y | | 6,12 | |
| A | FR-A-2 179 164  (MITSUBISHI CHEMICAL INDUSTRIES LTD) * Page 1, line 1 - page 3, lines 17,29-33; page 4, line 34 - page 5, line 31; page 6, line 39 - page 7, lines 6,37 - page 8, lines 5,15-40; examples 23,24; claims 6,8 * | 1,7 | |
| A | FR-A-2 187 739  (MITSUBISHI CHEMICAL INDUSTRIES LTD) * Page 1, lines 1-4,21 - page 3, line 34; page 5, lines 9-39; examples 21-24 * | 1,6,7,12 | TECHNICAL FIELDS SEARCHED (Int. Cl.4) C 07 C C 10 M B 01 D |
| A | DE-A-2 500 010  (EXXON RESEARCH AND ENGINEERING CO.) * Page 1, line 1 - page 3, line 5; page 4, line 22 - page 5, line 27; page 6, lines 9-13; page 7, line 18 - page 8, line 25; page 9, lines 2-13,17 - page 14, line 9 * | 6,12 | |

-/-

The present search report has been drawn up for all claims

| Place of search | Date of completion of the search | Examiner |
|---|---|---|
| THE HAGUE | 09-03-1988 | FISCHER W.H.F. |

European Patent Office

**EUROPEAN SEARCH REPORT**

Application Number

EP 88 30 0092

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A- 941 077 (S.W. BRIGGS) <br> * Page 2, lines 27-35; page 3, lines 18-64 * <br> ----- | . | |
| | | | TECHNICAL FIELDS SEARCHED (Int. Cl.4) |
| | The present search report has been drawn up for all claims | | |

| Place of search | Date of completion of the search | Examiner |
|---|---|---|
| THE HAGUE | 09-03-1988 | FISCHER W.H.F. |